# EUROPÄISCHE PATENTSCHRIFT

(11) **EP 1 227 801 B1**
(45) Veröffentlichungstag und Bekanntmachung des Hinweises auf die Patenterteilung: **13.09.2006**
(21) Anmeldenummer: 00983105.8
(22) Anmeldetag: 03.11.2000
(51) Int. Cl.: A61K 9/24

(54) **MEHRSCHICHTIGE FILMFÖRMIGE ZUBEREITUNG AUS HYDROPHILEN POLYMEREN ZUR SCHNELLEN FREISETZUNG VON WIRKSTOFFEN**
MULTI-LAYER PREPARATION IN FILM FORM, CONSISTING OF HYDROPHILIC POLYMERS, FOR THE RAPID RELEASE OF ACTIVE INGREDIENTS
PREPARATION MULTICOUCHE, SE PRESENTANT SOUS FORME DE FILM, CONSTITUEE DE POLYMERES HYDROPHILES ET PERMETTANT UNE LIBERATION RAPIDE D'AGENTS ACTIFS

(30) Priorität: 11.11.1999 DE 19954245
(43) Veröffentlichungstag der Anmeldung: 07.08.2002
(73) Patentinhaber: LTS LOHMANN Therapie-Systeme AG, 56626 Andernach (DE)
(72) Erfinder: KRUMME, Markus, 56567 Neuwied (DE)
(74) Vertreter: Flaccus, Rolf-Dieter
(86) Internationale Anmeldenummer: PCT/EP2000/010856
(87) Internationale Veröffentlichungsnummer: WO 2001/034121

(56) Entgegenhaltungen:
- EP-A- 0 781 546
- WO-A-98/17251
- DE-A- 3 618 553
- US-A- 5 700 478
- DATABASE WPI Section Ch, Week 199203 Derwent Publications Ltd., London, GB; Class A96, AN 1992-021918 XP002168209 & JP 63 171565 A (SEKISUI CHEM IND CO LTD) , 15. Juli 1988 (1988-07-15)

## Beschreibung

Die Erfindung betrifft ein Verfahren zur Herstellung einer mehrschichtigen, filmförmigen Zubereitung aus hydrophilen Polymeren, welche geeignet ist, die darin enthaltenen Stoffe innerhalb kurzer Zeit an die Umgebung abzugeben. Insbesondere betrifft es Zubereitungen der genannten Art, deren Schichten pharmako-logisch wirksame Stoffe enthalten.

Bei wirkstoffabgebenden Systemen, insbesondere bei Arzneiformen, ist es im Hinblick auf bestimmte Anwendungsgebiete erforderlich, daß eine größere Menge eines pharmakologisch aktiven Stoffes innerhalb kurzer Zeit im menschlichen oder tierischen Organismus freigesetzt wird. Dies kann beispielsweise dadurch erreicht werden, daß bei der Formulierung der Arzneiform eine möglichst große Oberfläche im Vergleich zum Volumen angestrebt wird. Dadurch wird gemäß den Diffusionsgesetzen die Abgabe der Inhaltsstoffe an die Umgebung beschleunigt.

Jedoch sind der Oberflächenvergrößerung Grenzen gesetzt, da die stoffabgebende Zusammensetzung andererseits auch möglichst kompakt sein muß, um dem System ausreichende Festigkeit zu verleihen und um eine gute Handhabbarkeit zu ermöglichen. So kann z.B. bei oralen Darreichungsformen die Fläche nicht unbeschränkt ausgedehnt werden.

Die Forderung nach einer schnellen Wirkstofffreisetzung bei gleichzeitiger Kompaktheit wird durch filmförmige Darreichungsformen erfüllt, welche die Wirkstoffe in gelöster, emulgierter oder suspendierter Form enthalten. Aufgrund der relativ kleinen Ausdehnung in Richtung der Filmdicke sind die Diffusionswege kurz und damit auch die für die Auflösung benötigten Zeiten kurz, so daß hohe Freisetzungsgeschwindigkeiten erreicht werden können.

Es ist bekannt, derartige Filme aus hydrophilen Polymeren als filmbildendem Agens herzustellen, gegebenenfalls unter Zusatz von Hilfsstoffen zur Einstellung der physikochemischen Parameter des Films, der chemischen Stabilität und/oder des Geschmacks.
Solche Filme sind erhältlich durch Herstellen einer Lösung, welche die Polymermatrix in einem hydrophilen Lösungsmittel, meist Wasser, enthält. Diese Lösung wird, mit Wirkund Hilfsstoffen versetzt, auf eine Prozeßfolie aus Kunststoff oder Metall beschichtet. Durch anschließende Trocknung wird das Lösungsmittel entzogen, und die Polymermatrix bleibt als Film zurück.
Jedoch kann mit diesem Verfahren nur eine gewisse maximale Filmdicke erreicht werden, wobei die Trocknungskinetik den begrenzenden Faktor darstellt. Gerade für wäßrige Systeme ist, bedingt durch die relativ hohe Verdampfungswärme, die mögliche Beschichtungsgeschwindigkeit bei gekoppelten Beschichtungs- und Trocknungsmaschinen relativ niedrig, so daß die dabei erzielbare Filmdicke entsprechend begrenzt ist.
Solche relativ dünnen Filme bieten zwar den Vorteil, daß sie im Organismus relativ schnell aufgelöst werden und damit eine rasche Wirkstofffreisetzung bewirken. Da aber die Filmdicke herstellungsbedingt begrenzt ist, ergibt sich daraus auch eine Beschränkung hinsichtlich der Beladung mit Wirkstoffen. Deshalb sind einschichtige Filme mit dem Nachteil einer niedrigen maximal möglichen Gesamtdosis behaftet.

Falls Kombinationspräparate mit den genannten Eigenschaften benötigt werden, kommt bei diesen dünnen Filmen als weiteres mögliches Problem die chemische Unverträglichkeit von zwei oder mehreren Wirkstoffen, oder auch sonstiger Inhaltsstoffe, z.B. Aromastoffe, hinzu.

Grundsätzlich ist es zwar möglich, eine Mehrfachbeschichtung dadurch zu erreichen, daß man auf einen bereits vorhandenen wirkstoffhaltigen Film mehrmals weitere Schichten aus der gleichen filmbildenden Lösung nacheinander aufträgt, wobei nach jeder Auftragung eine Trocknung vorgenommen wird. In der Praxis führt dies aber dazu, daß die jeweils bereits vorhandenen Schichten durch die Lösungsmittel der nachfolgend aufgetragenen Polymerlösung angegriffen oder aufgelöst werden. Dies kann zum einen den Herstellungsprozeß stören, zum andern verhindert es die Bildung von echten Mehrschicht-Systemen, in welchen die einzelnen Schichten voneinander getrennt sind. Zwar könnte eine Auflösung der zuerst hergestellten Schicht dadurch verhindert werden, daß man dort bestimmte Kristallisationsprozesse induziert. Allerdings wäre dies mit einer unerwünschten Verminderung der Löslichkeit der Filmschichten in wäßrigen Lösungen verbunden, die sich wiederum negativ auf die Wirkstofffreisetzung auswirkt.

DE-A 36 18 553 beschreibt einen medizinischen Klebestreifen für die Mundschleimhaut, der eine wirkstofffreie Trägerschicht und zwei Medikamentenschichten enthalten kann. Bei der Herstellung wird eine Lösung, welche die Komponenten der ersten Medikamentenschicht in einem Lösungsmittel enthält, auf eine Unterlage beschichtet und getrocknet. Auf diese getrocknete Schicht wird eine weitere Lösung, welche die Komponenten der zweiten Medikamentenschicht in einem Lösungsmittel enthält, beschichtet und getrocknet. Für die Herstellung der einzelnen Schichten werden entweder dieselben oder unterschiedliche Lösemittel verwendet. Die Auswahl der für die Herstellung der wirkstoffhaltigen Schichten verwendeten Lösemittel unterliegt keinen Beschränkungen und bewirkt keine vorteilhaften Eigenschaften.

Aufgabe der vorliegenden Erfindung war es deshalb, eine Zusammensetzung für die Abgabe von pharmakologischen oder anderen Wirkstoffen bereitzustellen, welche bei kompakten Abmesungen eine hohe wirkstoffbeladung und zugleich eine schnelle Freisetzung am Applikationsort ermöglicht. Insbesondere soll eine solche Zusammensetzung in wäßrigen Medien, beispielsweise in Körperflüssigkeiten, leicht löslich sein. Zudem war gefordert, daß eine solche Zusammensetzung auch die gemeinsame Verabreichung von zueinander inkompatiblen Wirk- oder anderen Inhaltsstoffen ermöglicht. Ferner sollte ein wenig aufwendiges Verfahren aufgezeigt werden, durch welches die Zusammensetzung hergestellt werden kann.

Überraschenderweise gelingt die Lösung der Aufgabe mit einem Verfahren zur Herstellung einer mehrschichtigen filmförmigen Zubereitung gemäß Anspruch 1 und den Unteransprüchen, welche weitere nützliche Ausführungformen beschreiben.

Vorzugsweise werden bei einer mehrschichtigen filmförmigen Zubereitung aus hydrophilen Polymeren die benachbarten Schichten sich dadurch voneinander unterscheiden, daß jeweils eine Schicht in einem nicht-wäßrigen Lösungsmittel löslich ist, in welchem die jeweilige benachbarte Schicht nicht oder nur schwer löslich ist.

Ein solcher Aufbau ermöglicht ein Herstellungsverfahren, bei dem zuerst eine wirkstoffhaltige Filmschicht ("Basisfolie") aus in Lösung, z.B. in Wasser, vorliegenden hydrophilen Polymeren hergestellt wird und anschließend auf die bereits vorhandene Basisfolie eine weitere Lösung aus filmbildenden Polymeren aufgetragen wird, wobei die letztgenannten Polymere in einem nicht-wäßrigen Lösungsmittel gelöst sind, in welchem die zuerst hergestellte Basisfolie nicht oder nur schwer löslich ist.
Beispielsweise kann die Basisfolie aus einem Polymer oder Polymergemisch bestehen, welches wasserlöslich ist, während das Polymer oder Polymergemisch für die folgende, benachbarte Folienschicht in höheren Alkoholen oder in Chloroform löslich ist, wobei die Polymere der Basisschicht in diesen organischen Lösungsmitteln nicht löslich sind. Die Löslichkeit der Polymere der nachfolgend aufgetragenen Schicht in organischen Lösungsmitteln schließt aber nicht aus, daß diese auch eine gute Wasserlöslichkeit aufweisen.

Auf die beschriebene Weise wird ein Beschichten mit einer zweiten (oder mit weiteren) wirkstoffhaltigen Schicht auf eine bereits vorhandene Schicht ermöglicht, ohne daß die bereits vorhandene Schicht durch die zur Herstellung der benachbarten Filmschicht verwendeten Lösungsmittel angegriffen oder aufgelöst wird.
Wegen des damit ermöglichten mehrschichtigen Aufbaus kann die Dicke der einzelnen Schichten gering gehalten werden, mit dem Vorteil, daß die Trocknungszeiten bei der Herstellung verkürzt werden können.

Die erfindungsgemäß hergestellten mehrschichtigen Systeme ermöglichen eine Wirkstoffbeladung, die höher ist als bei einschichtigen Systemen, wobei die Gesamtbeladung proportional mit der Anzahl der vorhandenen Schichten wächst. Diese höhere Wirkstoffbeladung wird erreicht, ohne daß eine Vergrößerung der Filmoberfläche und ein Verlust an Kompaktheit in Kauf genommen werden muß.
Der mehrschichtige Aufbau ermöglicht es ferner, daß innerhalb derselben Zubereitung, aber in verschiedenen Schichten, Wirkstoffe oder andere freisetzbare Inhaltsstoffe vorhanden sind, welche chemisch nicht miteinander kompatibel sind. Auf diese Weise können miteinander nicht verträgliche Wirkstoffe, Aromastoffe oder andere Inhaltsstoffe räumlich voneinander getrennt werden und es können Kombinationspräparate bereitgestellt werden, die sonst mit einer filmartigen Darreichungsform nicht möglich wären. Auch läßt sich durch eine Verteilung unverträglicher Inhaltsstoffe auf verschiedene Filmschichten die Stabilität solcher Arzneiformen verbessern.

Da die erfindungsgemäß hergesteltten filmförmigen mehrschichtigen Zubereitung ein günstiges Verhältnis von Oberfläche und Volumen aufweisen, kann eine schnelle Wirkstofffreisetzung in eine flüssige Umgebung auf dem Diffusionsweg erfolgen.

Besonders vorteilhaft sind Ausführungsformen welche sich dadurch auszeichnen, daß die Filmschichten in Wasser oder wäßrigen Systemen, vorzugsweise in Körperflüssigkeiten von Mensch oder Tier, löslich sind, unabhängig davon, daß sie auch in nicht-wäßrigen Systemen löslich sein können. Auf diese Weise läßt sich die Freisetzungsgeschwindigkeit insbesondere unter physiologischen Bedingungen weiter steigern. Bei mehrschichtigem Aufbau ist zwar die Auflösungszeit länger als bei einschichtigen Systemen, jedoch ist sie bei geeigneter Formulierung noch ausreichend kurz, um nicht den geschwindigkeitsbestimmenden Schritt bei der Wirkstofffreisetzung darzustellen.

Eine schnelle Auflösung der Zubereitung kann auch aus anderen Gründen erwünscht sein, beispielsweise wenn die Zubereitung zur Herstellung von Arzneiformen verwendet wird, die im Mundraum angewandt werden.
Dies kann erreicht werden durch die Auswahl solcher filmbildender Polymere, die einerseits wasserlöslich, andererseits aber auch in nicht-wäßrigen oder organischen Lösungsmitteln löslich sind, sofern die letztgenannte Eigenschaft benötigt wird, um bei der Herstellung einer weiteren Schicht der mehrschichtigen Zubereitung eine Auflösung einer bereits erzeugten Filmschicht zu verhindern.

Die erreichbare Freisetzungsgeschwindigkeit ist gemäß einer Ausführungsvariante der Erfindung dadurch gekennzeichnet, daß für die Auflösung einer Zubereitung mit einer Fläche von maximal 10 cm² in physiologischen Flüssigkeiten, oder künstlichen Nachbildungen solcher Flüssigkeiten, ein Zeitraum von höchstens 15 min, vorzugsweise von höchstens 5 min, besonders bevorzugt von höchstens 1 min benötigt wird.

Im Hinblick auf eine mögliche Verwendung der erfindungsgemäß hergestellten Zubereitungen zur Verabreichung von pharmakologisch wirksamen Substanzen an Menschen oder Tiere ist es vorteilhaft, wenn sie es aufgrund ihres Aufbaus ermöglicht, daß die Freisetzung der Substanzen aus den Filmschichten durch Auflösung dieser Schichten durch physiologische Flüssigkeiten oder künstliche Nachbildungen davon, vorzugsweise Körperflüssigkeiten von Mensch oder Tier, bewirkt wird. Dies kann durch die Auswahl geeigneter wasserlöslicher filmbildender Polymere und anderer löslicher Zusatzstoffe geschehen, wobei die geeigneten Polymere oder Zusatzstoffe dem Fachmann bekannt sind.

Die Polymerschichten der erfindungsgemäß hergesteltten filmförmigen Zubereitung eignen sich als Matrix zur Aufnahme und späteren Freisetzung von Inhaltsstoffen verschiedenster Art. Vorzugsweise kommen dabei Substanzen zum Einsatz, die ausgewählt sind aus der Gruppe, welche pharmakologische Wirkstoffe, Stoffe mit erfrischender Wirkung, Geschmacksstoffe, Aromastoffe und Süßstoffe umfaßt. Diese Substanzen können in einer bestimmten Schicht jeweils einzeln oder in Kombinationen vorliegen, wobei die Verträglichkeit der Stoffe untereinander zu beachten ist.
Die Filmschichten enthalten den oder die Wirkstoffe oder sonstigen Inhaltsstoffe in gelöster, emulgierter oder suspendierter Form.

Besonders bevorzugt sind Ausführungsformen die in mindestens einer der Filmschichten mindesten einen pharmakologischen Wirkstoff enthalten. Ebenso können eine oder mehrere Schichten auch Wirkstoffkombinationen enthalten.
Mit Vorteil können aber auch solche varianten der erfindungsgemäß hergestellten Zubereitungen verwendet werden, welche in mindestens zwei Schichten voneinander verschiedene Substanzen oder Wirkstoffe enthalten. Auf diese Weise lassen sich Kombinationspräparate formulieren, welche zueinander inkompatible Substanzen enthalten.

Der Schichtaufbau kann in weiten Bereichen variabel gestaltet werden. So sind einerseits Verbunde möglich, bei denen ausgehend von einer Basisschicht einseitig nacheinander weitere Schichten auf die beschrieben Weise aufgetragen werden. Andererseits ist auch ein symmetrischer Aufbau möglich, bei welchem an beide Seiten (Ober- und Unterseite) einer Basisschicht je eine weitere Schicht aufgebracht wird, sowie gegebenenfalls weitere Schichten.

Bei einer besonders bevorzugten Ausführungsform ist die Zusammensetzung aus zwei wasserlöslichen Schichten aufgebaut, wobei eine Schicht zusätzlich in einem organischen Lösungsmittel oder einem Gemisch solcher Lösungsmittel löslich ist, in welchem die andere Schicht nicht löslich ist.

Eine weitere besonders bevorzugte Zusammensetzung weist einen Aufbau aus drei wasserlöslichen Filmschichten auf. Dabei sind die beiden äußeren Schichten zusätzlich in einem organischen Lösungsmittel oder einem Gemisch solcher Lösungsmittel löslich sind, in welchem die mittlere Schicht nicht löslich ist. Die mittlere Schicht entspricht hier - hinsichtlich der Reihenfolge bei der Herstellung - der Basisschicht.

Als hydrophile filmbildende Polymere zur Herstellung der mehrschichtigen filmförmigen Zusammensetzungen kommen vor allem solche mit hoher Wasserlöslichkeit in Frage, vor allem verschiedene Cellulose-Ether, Polyvinylalkohol, Polyvinylacetat, Polyvinylpyrrolidon, ferner Copolymerisate aus den genannten Polymeren, sowie Alginate, Gelatine und diverse andere, dem Fachmann bekannte Stoffe, welche synthetischen, halbsynthetischen oder natürlichen Ursprungs sein können.

Besonders bevorzugt sind Zusammensetzungen, bei denen die Basisschicht aus Polyvinylalkohol oder Gemischen davon aufgebaut ist, beispielsweise aus einem teilhydrolysierten Polyvinylalkohol niedriger Viskosität (Mowiol® 8-88, Fa. Clariant). Polyvinylalkohole sind in Wasser gut löslich, nicht aber beispielsweise in Ethanol. Das letztgenannte Lösemittel kann deshalb erfindungsgemäß verwendet werden, um eine weitere Filmschicht auf diese Basisschicht aus Polyvinylalkohol aufzubringen.

Des weiteren sind Ausführungsformen der Erfindung bevorzugt, bei denen für die Herstellung der Basisschicht Cellulose-Ether, bevorzugt Hydroxymethylpropylcellulose, oder Gemische von Cellulose-Ethern, verwendet werden. Abgesehen von der genannten Verwendung bei der Erzeugung der Basisschicht können - bei entsprechend anderer Reihenfolge bei der Herstellung der mehrschichtigen Zubereitungen - Polyvinylalkohole oder Cellulose-Ether auch zur Herstellung der folgenden (benachbarten) Schichten verwendet werden.

In weiteren bevorzugten Ausführungsformen wird zur Erzeugung einer oder mehrerer weiterer Filmschichten der erfindungsgemäßen mehrschichtigen Zubereitung Polyvinylpyrrolidon als hydrophiles, wasserlösliches filmbildendes Polymer verwendet, beispielsweise Kollidon® (Fa. BASF).

Die Eigenschaften der erfindungsgemäß hergesteltten Zubereitungen können durch Hinzufügen von Hilfs- oder Zusatzstoffe optimiert und an die jeweiligen Erfordernisse angepaßt werden. Hierzu gehören z.B. Weichmacher, Pigmente, Zerfallsförderer, Netzmittel, resorptions- oder permeationsfördernde Substanzen, Strukturgeber und Texturmodifizierer, wobei dem Fachmann die Auswahl der geeigneten Zusätze bekannt ist.
Die mehrschichtigen Filmzubereitungen weisen vorzugsweise eine Dicke von mindestens 100 µm auf, besonders bevorzugt eine Dicke von mindestens 150 µm.

Die Herstellung der mehrschichtigen filmförmigen Zubereitungen kann mit den erfindungsgemäß vorgeschlagenen Verfahren auf einfache, zeitsparende und kostengünstige Weise erfolgen.
Hierzu wird zunächst eine Polymerlösung hergestellt, welche die gewünschten Wirkstoffe, gegebenenfalls zusammen mit Hilfsstoffen, enthält. Diese Lösung wird auf eine inerte Prozeßfolie aus Kunststoff oder Metall beschichtet, was mittels Rakel-, Walzenauftrags- oder Sprühverfahren erfolgen kann. Anschließend erhält man durch Trocknung einen Primärfilm, auch Basisschicht genannt.
Auf diese Basisschicht wird unter Verwendung der gleichen Verfahren im nächsten Schritt eine weitere wirkstoffhaltigen Polymerlösung aufgetragen, wobei für die Herstellung dieser Polymerlösung ein Lösemittel oder Lösemittelgemisch verwendet wird, welches die Primärschicht nicht angreift oder auflöst. Anschließend wird erneut getrocknet, und man erhält einen Zweischichtenverbund.
Nachfolgend können auf diesen Zweischichtenverbund eine oder mehrere weitere Schichten aufgetragen werden, unter Beachtung der erfindungsgemäßen Lehre in Bezug auf die Auswahl der Lösemittel. Eine weitere Möglichkeit ergibt sich, wenn man den Zweischichtenverbund von der Prozeßfolie ablöst, worauf die bislang noch unbeschichtete Unterseite des Primärfilms ebenfalls auf die beschriebene Weise mit einem Polymerfilm beschichtet werden kann, so daß man einen Dreischichtenverbund erhält. Dabei kann die hinzugefügte dritte Schicht entweder die gleiche Zusammensetzung aufweisen wie die zweite, so daß ein symmetrischer Aufbau resultiert, oder eine andere. In jedem Fall müssen aber das Polymer oder die Polymere dieser dritten Schicht gemäß der Lehre der Erfindung in einem Lösemittel oder -gemisch vorliegen, welches die Primärschicht nicht angreift oder auflöst.

Nach einem weiteren erfindungsgemäßen Herstellungsverfahren erfolgt die Herstellung der ersten Schicht ausgehend von einer Polymerschmelze, welche die gewünschten Wirkstoffe und gegebenenfalls Hilfs- oder Zusatzstoffe enthält. Die Auftragung der Schmelze auf eine inerte Prozeßfolie oder sonstige inerte Unterlage wird vorzugsweise durch Rakel-, Walzenauftrags-, Sprüh- oder Extrusionsverfahren bewerkstelligt. Anschließend erhält man daraus durch Kühlung und oder Trocknung einen Primärfilm (auch Basisschicht genannt).
Die Beschichtung dieses Primärfilms mit weiteren wirkstoffhaltigen Polymerfilmen zur Erzeugung einer mehrschichtigen Zubereitung erfolgt ausgehend von Polymerlösungen auf die bereits beschriebene Weise, wobei auch in diesem Fall bei der Auswahl der Polymere bzw. Lösemittel zu beachten ist, daß diese die Primärschicht bzw. gegebenenfalls die anderen bereits vorhandenen Filmschichten nicht angreifen oder auflösen dürfen.

Die erfindungsgemäßen Verfahren können sowohl kontinuierlich als auch diskontinuierlich durchgeführt werden. Für kontinuierliche Verfahren werden als inerte Unterlagen vorzugsweise Prozeßfolien oder Metallfolien oder -bänder verwendet, hingegen können bei diskontinuierlichen Verfahren grundsätzlich beliebige inerte Unterlagen zum Einsatz kommen.
Die erfindungsgemäß hergestellten Zubereitungen eignen sich vorzugsweise zur Abgabe von pharmazeutischen Wirkstoffen in menschliche oder tierische Körperflüssigkeiten, oder auch künstliche Nachbildungen davon. Insbesondere können sie vorteilhaft verwendet werden, um pharmakologische Wirkstoffe oder andere Substanzen im Mund-, Nasen oder Rachenraum oder sonstigen Körperöffnungen oder Körperhohlräumen des menschlichen oder tierischen Organismus zu applizieren, wobei die orale Verabreichung besonders bevorzugt ist.
Ferner ergibt sich eine weitere vorteilhafte Verwendung der erfindungsgemäß hergestellten Zubereitungen daraus, daß diese zur Herstellung von Arzneizubereitungen zur medikamentösen Therapie oder Prophylaxe von Krankheiten beim Menschen oder bei Tieren geeignet sind, vorzugsweise von Arzneizubereitungen zur oralen Verabreichung.

Zur Erläuterung der Erfindung wird folgendes Beispiel vorgestellt:

### Beispiel

Ein teilhydrolysierter Polyvinylalkohol niedriger Viskosität ((Mowiol® 8-88, Fa. Clariant) wird in heißem Wasser gelöst. Durch Zusatz geeigneter Hilfsstoffe wie Weichmachern, Pigmenten, Zerfallsförderern und ähnlichen dem Fachmann bekannten Additiven sowie den Wirkstoffen stellt man daraus eine viskose Masse her, die auf eine inerte Unterlage beschichtet wird. Nach Trocknung erhält man einen gut handhabbaren Primärfilm A, der in Wasser leicht löslich ist. Der getrocknete Primärfilm weist folgende Zusammensetzung auf:
28,6 Gew.-% Mowiol® 8-88,
7.9 Gew.-% Titandioxid,
37,2 Gew.-% Siliciumdioxid,
11,5 Gew.-% Polyethylenglykol 400,
4,6 Gew.-% Polyethylenglykol 4000 und
10,2 Gew.-% Sorbitol.

Anschließend stellt man eine zweite Masse für die zweite Schicht B basierend auf Polyvinylpyrrolidon (Kollidon® ; Fa. BASF) her, wobei das filmbildende Polymer in Ethanol gelöst wird. Diese zweite Masse enthält ebenfalls Wirkstoff(e) und gegebenenfalls geeignete Hilfsstoffe ähnlich der Zusammensetzung der ersten Schicht.

Die getrocknete Schicht B besitzt folgende Zusammensetzung:
32,8 Gew.-% Polyvinylpyrrolidon,
11,5 Gew.-% Hydroxypropylcellulose,
6,6 Gew.-% Titandioxid,
32,8 Gew.-% Siliciumdioxid und
16,4 Gew.-% Polyethylenglykol.

Der Primärfilm aus Mowiol® ist in dem für das Polymer der zweiten Schicht B gewählten Lösemittel (Ethanol) nicht löslich, und verhält sich daher inert gegen diese zweite Masse, welche deshalb auf den Primärfilm aufgetragen werden kann. Wiederum wird getrocknet, und man erhält einen in wäßrigen Lösungsmittelsystemen löslichen zweischichtigen Filmverbund mit der Schichtfolge A-B, da beide Schichtkomponenten wasserlöslich sind.

Nach Abziehen von der inerten Unterlage kann diese zweischichtige Zubereitung wahlweise an der noch freien Unterseite des Primärfilms mit einem weiteren Film beschichtet werden. Hierzu kann ebenfalls die zur Herstellung der Schicht B verwendete Polymerlösung verwendet werden, da diese die Primärschicht nicht auflöst. Man erhält auf diese Weise einen Dreischichtenverbund mit der Schichtfolge B-AB. Auch diese dreischichtige Zubereitung ist gut in wäßrigen Systemen löslich. Insgesamt kann in einem solchen Dreischichtsystem die dreifache Wirkstoffmenge verabreicht werden, bezogen auf die maximal mögliche Wirkstoffmenge, mit der eine einschichtige Filmzubereitung beladen werden kann.
Unter in vivo-Bedingungen können bei Filmabschnitten mit einer Größe von 15 x 15 mm durch geeignete Formulierungen Auflösungszeiten erreicht werden, welche bei 60 s oder darunter liegen.

## Patentansprüche

1. Verfahren zur Herstellung einer mehrschichtigen filmförmige Zubereitung aus hydrophilen Polymeren zur schnellen Freisetzung von in den Filmschichten enthaltenen Substanzen in eine flüssige Umgebung, **gekennzeichnet durch** folgende Schritte:
- Herstellung einer Polymerlösung oder einer Polymerschmelze, welche Wirkstoff(e) enthält;
- Beschichten der Polymerlösung oder Polymerschmelze auf eine inerte Unterlage, vorzugsweise **durch** Rakel-, Walzenauftrags-, Sprüh- oder Extrusionsverfahren, und nachfolgende Trocknung bzw. Kühlung und/oder Trocknung zur Erzeugung eines Primärfilms;
- Herstellung einer weiteren wirkstoffhaltigen Polymerlösung in einem Lösemittel oder Lösemittelgemisch, welches die Primärschicht nicht auflöst;
- Beschichtung dieser Lösung auf eine oder beide Seiten des Primärfilms, vorzugsweise **durch** Rakel-, Walzenauftrags-oder Sprühverfahren, und nachfolgende Trocknung;
- Beschichtung des Filmverbunds mit einer nächsten Schicht oder mit weiteren Schichten auf analoge Weise unter Verwendung von Lösemitteln, welche die jeweils zuvor erzeugte(n) Schicht(en) nicht auflösen.

2. Verfahren nach Anspruch 1 oder 2, **dadurch gekennzeichnet, daß** die Filmschichten aus in Wasser oder wäßrigen Systemen, vorzugsweise in Körperflüssigkeiten von Mensch oder Tier, löslichen Polymeren bestehen, unabhängig davon, daß sie auch in nicht-wäßrigen Systemen löslich sein können.

3. **Verfahren nach Anspruch 1 oder 2, dadurch gekennzeichnet, daß** die aus den Filmschichten freisetzbaren Substanzen ausgewählt sind aus der Gruppe, welche pharmakologische wirkstoffe, Stoffe mit erfrischender Wirkung, Geschmacksstoffe, Aromastoffe und Süßstoffe umfaßt.

4. Verfahren nach einem der Ansprüche 1 bis 3, **dadurch gekennzeichnet, daß** Polymere für die Herstellung der Filmschichten verwender werden, bei denen die Freisetzung der Substanzen aus den Filmschichten durch Auflösung dieser Schichten durch physiologische Flüssigkeiten Flüssigkeiten oder kunstliche Nachbildungen davon, vorzugsweise Körperflüssigkeiten von Mensch oder Tier, bewirkt wird.

5. Verfahren nach einem oder mehreren der vorangehenden Ansprüche, **dadurch gekennzeichnet, daß** Polymere für die Herstellung der Filmschichten verwendet werden, bei denen für die Auflösung einer Zubereitung mit einer Fläche von maximal 10 cm² in physiologischen Flüssigkeiten, oder künstlichen Nachbildungen solcher Flüssigkeiten, ein Zeitraum von höchstens 15 min, vorzugsweise von höchstens 5 min, besonders bevorzugt von höchstens 1 min benötigt wird.

6. Verfahren nach einem oder mehreren der vorangehenden Ansprüche, **dadurch gekennzeichnet, daß** mindestens eine Schicht mindestens einen pharmakologisch wirksamen Stoff enthält.

7. Verfahren nach einem oder mehreren der vorangehenden Ansprüche, **dadurch gekennzeichnet, daß** mindestens zwei Schichten dieselbe Substanz oder denselben pharmakologischen Wirkstoff, oder dieselbe Mischung dieser Stoffe, zur Freisetzung enthalten.

8. Verfahren nach einem oder mehreren der vorangehenden Ansprüche, **dadurch gekennzeichnet, daß** mindestens zwei Schichten freisetzbare Substanzen oder pharmakologische Wirkstoffe enthalten, die voneinander verschieden sind.

9. Verfahren nach einem oder mehreren der vorangehenden Ansprüche, **dadurch gekennzeichnet, daß** die Zubereitung aus zwei wasserlöslichen Schichten aufgebaut wird, wobei eine Schicht zusätzlich in einem organischen Lösungsmittel oder einem Gemisch solcher Lösungsmittel löslich ist, in welchem die anderer Schicht nicht löslich ist.

10. Verfahren nach einem oder mehreren der vorangehenden Ansprüche, **dadurch gekennzeichnet, daß** sie aus drei wasserlöslichen Schichten aufgebaut wird, wobei die beiden äußeren Schichten zusätzlich in einem organischen Lösungsmittel oder einem Gemisch solcher Lösungsmittel löslich sind, in welchem die mittlere Schicht nicht löslich ist.

11. Verfahren nach einem oder mehreren der vorangehenden Ansprüche, **dadurch gekennzeichnet, daß** die hydrophilen Polymere der Filmschichten eine hohe Wasserlöslichkeit aufweisen und ausgewählt sind aus der Gruppe, welche Cellulose-Ether, Polyvinylalkohol, Polyvinylacetat, Polyvinylpyrrolidon, ferner Copolymerisate aus den genannten Polymeren, sowie Alginate, Gelatine und andere Stoffe synthetischen, halbsynthetischen oder natürlichen Ursprungs umfaßt.

12. Verfahren nach einem oder mehreren der vorangehenden Ansprüche, **dadurch gekennzeichnet, daß** für eine von jeweils zwei benachbarten Filmschichten, vorzugsweise die zuerst hergestellte Schicht, Polyvinylalkohol oder Gemische davon verwendet werden.

13. Verfahren nach einem oder mehreren der vorangehenden Ansprüche, **dadurch gekennzeichnet, daß** für eine von jeweils zwei benachbarten Filmschichten, vorzugsweise die zuerst hergestellte Schicht, Cellulose-Ether, bevorzugt Hydroxymethylpropylcellulose, oder Gemische von Cellulose-Ethern, verwendet werden.

14. Verfahren nach einem oder mehreren der vorangehenden Ansprüche, **dadurch gekennzeichnet, daß** für eine von jeweils zwei benachbarten Filmschichten Polyvinylpyrrolidon oder Gemische davon verwendet werden.

15. Verfahren nach Anspruch 9 oder 10, **dadurch gekennzeichnet, daß** für eine der Schichten Polymere verwendet werden, die ausgewählt sind aus der Gruppe, die Polyvinylalkohol-oder Gemische von Polyvinylalkoholen - und Celluloseether, bevorzugt Hydroxymethylpropylcellulose - oder Gemische von Cellulose-Ethern - umfaßt, und daß für mindestens eine weitere Schicht Polyvinylpyrrolidon oder Gemische davon verwendet werden.

16. Verfahren nach einem oder mehreren der vorhergehenden Ansprüche, **dadurch gekennzeichnet, daß** für mindestens eine weitere Schicht (a) Cellulose-Ether, bevorzugt Hydroxymethylpropylcellulose, oder Gemische von Cellulose-Ethern, oder (b) Polyvinylpyrrolidon oder Gemische davon oder (c) Polyvinylalkohol oder Gemische davon verwendet werden.

17. Verfahren nach einem oder mehreren der vorhergehenden Ansprüche, **dadurch gekennzeichnet, daß** für mindestens eine der Schichten Hilfs- oder Zusatzstoffe verwendet werden, die ausgewählt sind aus der Gruppe, welche Weichmacher, Pigmente, Zerfallsförderer, Netzmittel, resorptions- oder permeationsfördernde Substanzen, Strukturgeber und Texturmodifizierer umfaßt.

## Claims

1. Process for the production of a multilayer, film-shaped preparation of hydrophilic polymers for rapid release of substances present in the film layers into liquid surroundings, **characterized by** the following steps:
- production of a polymer solution or a polymer melt which contains active ingredient(s);
- coating of the polymer solution or polymer melt onto an inert substrate, preferably by knife or roller application, spraying processes or extrusion processes and subsequent drying, or cooling and/or drying, to produce an initial film;
- production of another active ingredient-containing polymer solution in a solvent or solvent mixture which does not dissolve the initial layer;
- coating of this solution onto one or both sides of the initial film, preferably by knife or roller application or spraying processes, and subsequent drying;
- coating of the film composite with a next layer or with further layers in an analogous way using solvents which do not dissolve the layer(s) produced before in each case.

2. Process according to claim 1, **characterized in that** the film layers consist of polymers that are soluble in water or aqueous systems, preferably in human or animal body fluids, irrespective of the possibility that they are also soluble in nonaqueous systems.

3. Process according to Claim 1 or 2, **characterized in that** the substances which can be released from the film layers are selected from the group which comprises pharmacological active ingredients, substances with refreshing effect, flavorings, odorizers and sweeteners.

4. Process according to any of Claims 1 to 3, **characterized in that** such polymers are used for the production of the film layers where the release of the substances from the film layers is brought about by dissolution of these layers by physiological liquids or artificial simulations thereof, preferably human or animal body fluids.

5. Process according to one or more of the preceding claims, **characterized in that** such polymers are used for the production of the film layers where the time required for dissolution of a preparation with an area not exceeding 10 cm² in physiological fluids, or artificial simulations of such fluids, does not exceed 15 min, preferably does not exceed 5 min, particularly preferably does not exceed 1 min.

6. Process according to one or more of the preceding claims, **characterized in that** at least one layer contains at least one pharmacologically active substance.

7. Process according to one or more of the preceding claims, **characterized in that** at least two layers contain the same substance or the same pharmacological active ingredient, or the same mixture of these substances, for the release.

8. Process according to one or more of the preceding claims, **characterized in that** at least two layers contain releasable substances or pharmacological active ingredients which are different from one another.

9. Process according to one or more of the preceding claims, **characterized in that** the preparation is built up of two water-soluble layers, with one layer additionally being soluble in an organic solvent, or a mixture of such solvents, in which the other layer is insoluble.

10. Process according to one or more of the preceding claims, **characterized in that** the preparation is built up of three water-soluble layers, with the two outer layers additionally being soluble in an organic solvent, or a mixture of such solvents, in which the middle layer is insoluble.

11. Process according to one or more of the preceding claims, **characterized in that** the hydrophilic polymers of the film layers have a high solubility in water and are selected from the group which comprises cellulose ethers, polyvinyl alcohol, polyvinyl acetate, polyvinylpyrrolidone, also copolymers of the polymers mentioned, and alginates, gelatin and other substances of synthetic, semisynthetic or natural origin.

12. Process according to one or more of the preceding claims, **characterized in that** for one of in each case two adjacent film layers, preferably the layer which is produced first, polyvinyl alcohol or mixtures thereof is/are used.

13. Process according to one or more of the preceding claims, **characterized in that** for one of in each case two adjacent film layers, preferably the layer which is produced first, cellulose ethers, preferably hydroxymethylpropylcellulose, or mixtures of cellulose ethers are used.

14. Process according to one or more of the preceding claims, **characterized in that** for one of in each case two adjacent film layers polyvinylpyrrolidone or mixtures thereof is/are used.

15. Process according to claim 9 or 10, **characterized in that** for one of the layers polymers which are selected from the group which comprises polyvinyl alcohol - or mixtures of polyvinyl alcohols - and cellulose ethers, preferably hydroxymethylpropylcellulose - or mixtures of cellulose ethers - are used, and **in that** for at least one further layer polyvinylpyrrolidone or mixtures thereof is/are used.

16. Process according to one or more of the preceding claims, **characterized in that** for at least one further layer (a) cellulose ethers, preferably hydroxymethylpropylcellulose, or mixtures of cellulose ethers, or (b) polyvinylpyrrolidone or mixtures thereof or (c) polyvinyl alcohol or mixtures thereof is/are used.

17. Process according to one or more of the preceding claims, **characterized in that** for at least one of the layers auxiliaries or additives are used which are selected from the group comprising plasticizers, pigments, disintegration promoters, wetting agents, absorption- or permeation-promoting substances, structuring agents and texture modifiers.

## Revendications

1. Procédé de fabrication d'une préparation pelliculaire multicouche en polymères hydrophiles en vue de la libération rapide de substances contenues dans les couches pelliculaires dans un environnement liquide, **caractérisé par** les étapes suivantes :
- production d'une solution de polymère ou d'une masse fondue de polymère qui contient une ou plusieurs substances actives ;
- dépôt de la solution de polymère ou de la masse fondue de polymère sur une sous-couche inerte, de préférence au moyen d'un procédé d'application par raclette, par cylindre, par pulvérisation ou par extrusion, puis séchage, respectivement refroidissement et/ou séchage pour produire une pellicule primaire ;
- production d'une autre solution de polymère contenant une substance active dans un solvant ou dans un mélange de solvants qui ne dissout pas la couche primaire ;
- dépôt de cette solution sur un côté, ou les deux, de la pellicule primaire, de préférence au moyen d'un procédé d'application par raclette, par cylindre ou par pulvérisation, puis séchage ;
- revêtement du composite pelliculaire avec une couche ultérieure ou avec d'autres couches de manière analogue en utilisant des solvants qui ne dissolvent pas à chaque fois la ou les couches antérieurement déposées.

2. Procédé selon la revendication 1 ou 2, **caractérisé en ce que** les couches pelliculaires se composent de polymères solubles dans l'eau ou des systèmes aqueux, de préférence dans les liquides corporels de l'homme ou des animaux, indépendamment du fait qu'ils peuvent être également solubles dans des systèmes non aqueux.

3. Procédé selon la revendication 1 ou 2, **caractérisé en ce que** les substances libérables à partir des couches pelliculaires sont choisies dans le groupe qui comprend les substances actives pharmacologiques, les substances à action rafraîchissantes, les exhausteurs de goût, les substances aromatiques et les édulcorants.

4. Procédé selon l'une des revendications 1 à 3, **caractérisé en ce qu'**on utilise des polymères pour la production des couches pelliculaires, où la libération des substances à partir des couches pelliculaires est produite par la dissolution de ces couches par des liquides physiologiques ou des imitations artificielles de ces liquides, de préférence des liquides corporels de l'homme ou des animaux.

5. Procédé selon une ou plusieurs des revendications précédentes, **caractérisé en ce qu'**on utilise des polymères pour la production des couches pelliculaires, où pour la dissolution d'une préparation d'une surface d'au maximum 10 cm² dans des liquides physiologiques, ou des imitations artificielles de tels liquides, on a besoin d'un délai d'au plus 15 minutes, de préférence d'au plus 5 minutes, de façon particulièrement préférée d'au plus 1 minute.

6. Procédé selon une ou plusieurs des revendications précédentes, **caractérisé en ce qu'**au moins une couche contient au moins une substance pharmacologiquement active.

7. Procédé selon une ou plusieurs des revendications précédentes, **caractérisé en ce qu'**au moins deux couches contiennent la même substance ou la même substance active pharmacologique, ou le même mélange de ces substances, aux fins de libération.

8. Procédé selon une ou plusieurs des revendications précédentes, **caractérisé en ce qu'**au moins deux couches contiennent des substances libérables ou des substances actives pharmacologiques qui sont différentes l'une de l'autre.

9. Procédé selon une ou plusieurs des revendications précédentes, **caractérisé en ce que** la préparation comprend de deux couches solubles dans l'eau, l'une des couches étant en outre soluble dans un solvant organique ou un mélange de tels solvants, dans lequel l'autre couche n'est pas soluble.

10. Procédé selon une ou plusieurs des revendications précédentes, **caractérisé en ce que** la préparation comprend de trois couches solubles dans l'eau, les deux couches externes étant en outre solubles dans un solvant organique ou dans un mélange de tels solvants, dans lequel la couche médiane n'est pas soluble.

11. Procédé selon une ou plusieurs des revendications précédentes, **caractérisé en ce que** les polymères hydrophiles des couches pelliculaires présentent une haute solubilité dans l'eau et sont choisis dans le groupe qui comprend les éthers de cellulose, l'alcool polyvinylique, l'acétate de polyvinyle, la polyvinylpyrrolidone, ou encore les copolymères des polymères mentionnés, ainsi que les alginates, les gélatines et d'autres substances d'origine synthétique, semi-synthétique ou naturelle.

12. Procédé selon une ou plusieurs des revendications précédentes, **caractérisé en ce que** pour l'une de deux couches pelliculaires adjacentes respectives, de préférence la première couche produite, on utilise l'alcool polyvinylique ou ses mélanges.

13. Procédé selon une ou plusieurs des revendications précédentes, **caractérisé en ce que** pour l'une de deux couches pelliculaires adjacentes respectives, de préférence la première couche produite, on utilise des éthers de cellulose, de préférence l'hydroxyméthylpropylcellulose, ou des mélanges d'éthers de cellulose.

14. Procédé selon une ou plusieurs des revendications précédentes, **caractérisé en ce que** pour l'une de deux couches pelliculaires adjacentes respectives, on utilise la polyvinylpyrrolidone ou ses mélanges.

15. Procédé selon la revendication 9 ou 10, **caractérisé en ce que** pour une des couches on utilise des polymères qui sont choisis dans le groupe qui comprend l'alcool polyvinylique ou des mélanges d'alcool polyvinyliques - et des éthers de cellulose, de préférence l'hydroxyméthylpropylcellulose - ou des mélanges d'éthers de cellulose, et **en ce que** pour au moins une autre couche on utilise la polyvinylpyrrolidone ou ses mélanges.

16. Procédé selon une ou plusieurs des revendications précédentes, **caractérisé en ce que** pour au moins une autre couche on utilise (a) des éthers de cellulose, de préférence l'hydroxyméthylpropylcellulose, ou des mélanges d'éthers de cellulose, ou (b) la poylvinylpyrrolidone ou ses mélanges ou (c) l'alcool polyvinylique ou ses mélanges.

17. Procédé selon une ou plusieurs des revendications précédentes, **caractérisé en ce que** pour au moins une des couches on utilise des adjuvants ou additifs qui sont choisis dans le groupe qui comprend les plastifiants, les pigments, les accélérateurs de décomposition, les agents mouillants, les substances favorisant la résorption ou la perméation, les agents structurants et les modificateurs de texture.
